Europäisches Patentamt

European Patent Office

Office européen des brevets

(19)

(11) Publication number: **0 240 334**
**A1**

(12)

# EUROPEAN PATENT APPLICATION

(21) Application number: **87302832.8**

(22) Date of filing: **01.04.87**

(51) Int. Cl.⁴: **C 12 N 15/00**
**C 12 N 9/64, A 61 K 37/54**

(30) Priority: **02.04.86 GB 8608014**
**10.12.86 GB 8629483**

(43) Date of publication of application:
**07.10.87 Bulletin 87/41**

(84) Designated Contracting States:
**BE CH DE ES FR GB GR IT LI LU NL SE**

(71) Applicant: **BEECHAM GROUP PLC**
**Beecham House Great West Road**
**Brentford Middlesex TW8 9BD (GB)**

(72) Inventor: **Browne, Michael Joseph**
**Beecham Pharm.§Great Burgh & Yew Tree Bottom**
**Epsom Surrey, KT18 5XQ (GB)**

(74) Representative: **Valentine, Jill Barbara et al**
**Beecham Pharmaceuticals Patents & Trade Marks Dept.**
**Great Burgh Yew Tree Bottom Road**
**Epsom Surrey KT18 5XQ (GB)**

(54) **Modified enzyme.**

(57) A fibrinolytically active tissue-type plasminogen activator which has been modified in the region of amino acids 67 to 69.

EP 0 240 334 A1

## Description

<u>Novel Compounds</u>

The present invention relates to a modified fibrinolytic enzyme in particular modified tissue-type plasminogen activator, its preparation, pharmaceutical compositions containing it and its use in the treatment of thrombotic disease.

The sequence of amino acids making up the enzyme tissue-type plasminogen activator (t-PA) and the nucleotide sequence for the cDNA which codes for t-PA are known (see Pennica et al, 1983; Nature, 301, 214). t-PA is known to have fibrinolytic activity.

It has been shown (Bányai, L. et al, 1983: FEBS Lett., 163, 37) that a part of the t-PA enzyme shows structural homology with human and murine epidermal growth factors. This region from amino acid residues 44 to 91 has been termed the "growth factor domain". The genetic information which codes for the major part of this domain, residues 51 to 86 inclusive, and partially codes for residues 50 and 87, lies on a single exon (Ny, T. et al, 1984; Proc. Nat. Acad. Sci. U.S.A., 81, 5355). Within this region there is a sequence of three amino acids:

```
          - tyr - phe - ser-
position 67      68      69
```

The applicants have now identified modified forms of the t-PA enzyme which retain fibrinolytic activity. According to the present invention there is provided a fibrinolytically active tissue-type plasminogen activator which has been modified in the region of amino acids 67 to 69.

Suitable modifications may include removal or deletion of certain amino acid residues, or replacement of one or more amino acid residues with different amino acid residues.

In a preferred aspect, the modification comprises the deletion of all or part of the region.

More particularly, the modification may comprise the deletion of residues 67 and 68 or 67 to 69, more preferably residues 67 to 69.

As used herein, the term tissue-type plasminogen activator denotes a plasminogen activator of the group having the immunological properties defined for t-PA at the XXVIII Meeting of the International Committee on Thrombosis and Heamostasis, Bergamo, Italy, 27 July 1982.

The amino acid sequence of various forms of t-PA are known. The abovementioned Nature 1983 reference discloses the sequence for the L-chain and the mature S-chain forms of t-PA, also discussed by Vehar et.al., Biotechnology, 1984 2, 1051-7 in which the processing of initially formed t-PA by removal of a pro-sequence to give the S-chain form is reported. Pohl et.al., FEBS letters, 1984, Vol. 168 No. 1, 29-32, refers to the N-terminal multiplicity of t-PA and discloses the U-chain form. The numbering system for the amino acid sequence of t-PA used herein is that described in the Nature 1983 reference for mature (S-chain) t-PA in which the N-terminal serine is numbered 1. By this system, L-chain t-PA has an N-terminal glycine residue at position -3 and U-chain t-PA has an N-terminal valine at position 4. It is understood that the tissue-type plasminogen activator modified in accordance with the present invention encompasses all such variant forms.

The modified t-PA of the invention may be derivatised to provide pharmaceutically useful conjugates analogous to known t-PA-containing conjugates, for example:

(a) an enzyme-protein conjugate as disclosed in EP-A-O 155 388, in which the catalytic site on the enzyme which is responsible for fibrinolytic activity is blocked by a human protein attached thereto by way of a reversible linking group;

(b) an enzyme-protein conjugate as disclosed in EP-A-O 152 736, comprising at least one optionally blocked fibrinolytic enzyme linked by way of a site other than the catalytic site responsible for fibrinolytic activity to at least one human protein;

(c) a protein-polymer conjugate as disclosed in EP-A-0 183 503 comprising a pharmaceutically useful protein linked to at least one water soluble polymer by means of a reversible linking group; or

(d) an enzyme conjugate as disclosed in EP-A-0 184 363 comprising a plurality of fibrinolytic enzymes linked together through the active centres thereof by means of a removable blocking group.

The modified t-PA of the invention may take the place of t-PA as the enzyme or (human) protein components, as appropriate, of any of the conjugates described above.

The modified t-PA of the invention or conjugate thereof can be further derivatised such that any catalytic site essential for fibrinolytic activity is optionally blocked by a removable blocking group.

The above mentioned derivatives of the modified t-PA may be used in any of the methods and compositions described hereinafter for the modified t-PA itself.

As used herein the expression "removable blocking group" includes groups which are removable by hydrolysis at a rate such that the pseudo-first order rate constant for hydrolysis is in the range of $10^{-6}$ sec$^{-1}$ to $10^{-2}$ sec$^{-1}$ in isotonic aqueous media at pH 7.4 at 37°C.

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$alkanoyloxy, $C_{1-6}$ alkanoylamino, amino or p-guanidino.

Suitable optional substituents for acryloyl blocking groups include $C_{1-6}$ alkyl, furyl, phenyl or $C_{1-6}$alkylphenyl.

In a further aspect, the invention provides a process for preparing modified tissue-type plasminogen activator according to the invention which process comprises expressing DNA encoding said modified tissue-type plasminogen activator in a recombinant host cell and recovering the modified tissue-type plasminogen activator product.

The modification of the t-PA can therefore be carried out by conventional genetic engineering techniques in which the cDNA which codes for t-PA is modified and the modified cDNA expressed in a prokaryote or eukaryote host.

The DNA polymer comprising a nucleotide sequence that encodes the modified t-PA also forms part of the invention.

The process of the invention may be performed by conventional recombinant techniques such as described in Maniatis et. al., Molecular Cloning - A Laboratory Manual; Cold Spring Harbor, 1982.

In particular, the process may comprise the steps of:

    i) preparing a replicable expression vector capable, in a host cell, of expressing a DNA polymer comprising a nucleotide sequence that encodes said modified tissue-type plasminogen activator;

    ii) transforming a host cell with said vector;

    iii)culturing said transformed host cell under conditions permitting expression of said DNA polymer to produce said modified tissue-type plasminogen activator; and

    iv) recovering said modified tissue-type plasminogen activator.

The invention also provides a process for preparing the DNA polymer by the condensation of appropriate mono-, di- or oligomeric nucleotide units.

The preparation may be carried out chemically, enzymatically, or by a combination of the two methods, in vitro or in vivo as appropriate. Thus, the DNA polymer may be prepared by the enzymatic ligation of chemically synthesised DNA fragments, by conventional methods such as those described by D. M. Roberts et al in Biochemistry 1985, 24, 5090-5098. The chemical synthesis may be performed generally as described hereinafter for the synthesis of an oligodeoxyribonucleotide. Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of $10°-37°C$, generally in a volume of 50µl or less. Enzymatic ligation of DNA fragments may be carried out using a DNA ligase such as T4 DNA ligase in an appropriate buffer at a temperature of 4°C to ambient, generally in a volume of 50µl or less. DNA polymer which encodes the modified t-PA may be prepared by site-directed mutagenesis of the cDNA which codes for tissue-type plasminogen activator, by conventional methods such as those described by G. Winter et al in Nature 1982, 299, 756-758 or by Zoller and Smith 1982; Nucl. Acids Res., 10, 6487-6500, or deletion mutagenesis such as described by Chan and Smith in Nucl. Acids Res., 1984, 12, 2407-2419 or by G. Winter et al in Biochem. Soc. Trans., 1984, 12, 224-225. Expression of the modified cDNA can be carried out by conventional methods.

The above described mutagenesis processes involve the use of oligodeoxyribonucleotides which can be designed according to the changes in the cDNA sequence coding for amino acids 67 to 69 inclusive required.

The oligodeoxyribonucleotides which may be used in such processes also form part of the invention.

The invention also provides a process for preparing an oligodeoxyribonucleotide of the invention, which process comprises the deprotection of a second oligodeoxyribonucleotide having the same sequence of bases as said oligodeoxyribonucleotide and in which all free hydroxy and amino groups are protected.

The oligodeoxyribonucleotides used in the process can be prepared by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982), or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society, 1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801.

Solid supports which may be employed include conventional supports known in the art, for example kieselguhr-polydimethylacrylamide, silica, controlled-pore glass, or cellulose paper discs. The base at the 3'-end of the oligodeoxyribonucleotide to be prepared is attached, via a spacer group connected to the 3'-oxygen of the terminal 2'-deoxyribose unit, to the solid support and assembly of the oligodeoxyribonucleotide (in a protected form) is carried out in the 3' → 5'-direction by cycles of synthesis, the required operations being performed either manually or by an automated instrument. At the end of the synthesis, the protected oligodeoxyribonucleotide may be cleaved from the solid support either before, after, or at the same time as other deprotection steps that are carried out once the desired sequence of bases has been assembled, as described hereinbelow. Conveniently the oligodeoxyribonucleotide is removed from the solid support by treatment with a basic reagent such as aqueous ammonia at ambient or slightly elevated temperature, or with 1,1,3,3 -tetramethyl-guanidinium-syn- 2-nitrobenzaldoximate or similar reagents in aqueous dioxan at ambient or slightly elevated temperature.

Alternatively, the preparation may be carried out conventionally by phosphotriester chemistry in solution as described, for example, by C.B. Reese, Tetrahedron, 1978, 34, 3143-3179.

The required sequence of nucleotide bases in the oligodeoxyribonucleotide may be built up conventionally, for example as described in the aforementioned publications, by the condensation of appropriate mono-, di- or oligomeric nucleotide units in the presence of a suitable condensing agent such as I-(mesitylenesulphonyl)-3-nitro-I,2,4-triazole in solvent such as pyridine at ambient or slightly elevated temperature (when phosphotriester chemistry is employed) or by a condensing agent such as tetrazole in acetonitrile at ambient temperature (when phosphite or phosphoramidite chemistry is used). Optionally a 'capping' step is introduced after each condensation step to inactivate any unreacted starting material containing a free 5′-hydroxy group. Such a capping step may suitably be carried out by an acylating agent such as acetic anhydride in 2,6-lutidine and 4-N,N-dimethylamino-pyridine in tetrahydrofuran at ambient temperature.

When phosphite or phosphoramidite chemistry is employed, the phosphorus (III) atom in the phosphite-triester internucleotide linkage created in each condensation step is oxidised, giving the corresponding phosphotriester linkage, before a further condensation step is carried out. Such oxidations may be carried out using a convenient oxidising agent, for example iodine in aqueous tetrahydrofuran in the presence of a base such as lutidine. The oxidation step is conveniently carried out after the optional 'capping' step as hereinbefore described.

During the synthesis of the oligodeoxyribonucleotide, the hydroxy groups in each of the internucleotide phosphodiester bridges may be protected by an aryl group or alkyl group conventionally employed for the purpose, for example 2- or 4-chlorophenyl, methyl, or 2-cyanoethyl, to prevent branching of the molecule. The aryl protecting groups are removed at the end of the synthesis by treatment with an agent conventionally employed for the purpose, for example I,I,3,3-tetramethyl-guanidinium- syn -2-nitrobenzaldoximate in an aqueous solvent such as aqueous dioxan at ambient temperature. The methyl groups may be removed at the end of the synthesis by treatment with triethylammonium thiophenolate in dioxan at ambient temperature. The 2-cyanoethyl groups may be removed at the end of the synthesis with a basic reagent such as triethylamine or tert-butylamine in pyridine at ambient temperature, or alternatively by treatment with concentrated aqueous ammonia at about 50°C (a step which simultaneously and advantageously removes the protecting groups present on the A, C and G bases as described hereinbelow).

The amino groups present in the A, C and G bases are protected by base-labile protecting groups, such as benzoyl for A and C, and isobutyryl for G. Such groups may be removed by treatment with basic reagents, for example ammonia at ambient or slightly elevated temperature, for example 50°C. The 5′-hydroxy group of the terminal ribose moiety is protected by an acid-labile group such as trityl, 4,4′-dimethoxytrityl or pixyl (9-phenyl-9-xanthyl), which is removed before each condensation step and at the end of synthesis. Such groups may be removed by treatment with an acidic reagent such as di- or trichloroacetic acid in an anhydrous solvent such as dichloromethane or chloroform, at ambient temperature.

The expression of the DNA polymer encoding the modified t-PA in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and combining said linear segment with one or more DNA molecules which, together with said linear segment, encode the modified t-PA, under ligating conditions.

The ligation of the linear segment and more than one DNA molecule may be carried out simultaneously or sequentially as desired.

Thus, the DNA polymer may be preformed or formed during the construction of the vector, as desired.

In particular, the replicable expression vector may be prepared by cleaving a vector compatible with the host cell to provide a linear DNA segment having an intact replicon, and combining said linear segment with the DNA polymer encoding the modified t-PA, under ligating conditions.

The choice of vector will be determined in part by the host cell, which may be prokaryotic or eukaryotic. Suitable vectors include plasmids, bacteriophages, recombinant viruses and cosmids.

The preparation of the replicable expression vector may be carried out conventionally with appropriate enzymes for restriction, polymerisation and ligation of the DNA, by procedures described in, for example, Maniatis et al cited above. Polymerisation and ligation may be performed as described above for the preparation of the DNA polymer. Digestion with restriction enzymes may be performed in an appropriate buffer at a temperature of 20°-70°C, generally in a volume of 50μl or less with 0.I-I0μg DNA.

The recombinant host cell is prepared, in accordance with the invention, by transforming a host cell with a replicable expression vector of the invention under transforming conditions. Suitable transforming conditions are conventional and are described in, for example, Maniatis et al cited above, or "DNA Cloning" Vol. II, D.M. Glover ed., IRL Press Ltd, 1985.

The choice of transforming conditions is determined by the host cell. Thus, a bacterial host such as E. coli may be treated with a solution of CaCl2 (Cohen et al, Proc. Nat. Acad. Sci., 1973 69, 2110) or with a solution comprising a mixture of RbCl, MnCl2, potassium acetate and glycerol, and then with 3-[N-morpholino]-propane-sulphonic acid, RbCl and glycerol. Mammalian cells in culture may be transformed by calcium co-precipitation of the vector DNA onto the cells. The invention also extends to a host cell transformed with a replicable expression vector of the invention.

Culturing the transformed host cell under conditions permitting expression of the DNA polymer is carried out conventionally, as described in, for example, Maniatis et al and "DNA Cloning" cited above. Thus, preferably the cell is supplied with nutrient and cultured at a temperature below 45°C.

The modified t-PA expression product is recovered by conventional methods according to the host cell. Thus, where the host cell is bacterial, such as E. coli it may be lysed physically, chemically or enzymatically and the protein product isolated from the resulting lysate. Where the host cell is mammalian, the product may generally be isolated from the nutrient medium. The DNA polymer may be assembled into vectors designed for isolation of stable transformed mammalian cell lines expressing the modified t-PA; e.g. bovine papillomavirus vectors (DNA cloning Vol.II D.M. Glover Ed. IRL press 1985; Kaufman, R.J. et al, Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al., European Patent Application No. 0093619, 1983).

It will be appreciated that, depending upon the host cell, the modified t-PA prepared in accordance with the invention may be glycosylated to varying degrees. Furthermore, as observed by Pohl et.al., Biochemistry, 1984, 23, 3701-3707, varying degrees of glycosylation may also be found in unmodified, naturally occurring t-PA. The modified t-PA of the invention is understood to include such glycosylated variations.

In a preferred embodiment, a modified tissue-type plasminogen activator enzyme is prepared by a process which comprises effecting a deletion mutagenesis upon the cDNA which codes for tissue-type plasminogen activator using an oligodeoxyribonucleotide comprising a nucleotide sequence which is complementary or corresponds to a region of the t-PA cDNA extending 5′ from nucleotide 387 inclusive, said sequence being joined directly to a nucleotide sequence which is complementary or corresponds to a region extending 3′ from (a) nucleotide 397 or (b) nucleotide 394 inclusive of t-PA cDNA and expressing the modified cDNA in a prokaryote or eurokaryote host.

The oligodeoxyribonucleotide employed in variant (a) of the preferred embodiment is suitably at least 16 nucleotides in length, preferably at least 24 nucleotides in length. Suitably it contains at least 8 and preferably at least 12 nucleotides which are complementary to nucleotides on each side of the deletion region.

A preferred oligodeoxyribonucleotide is of sequence (I).

5′d(GCACACGAAATCCAGGGCCTGCTG)3′ (I)

The 12 bases at the 5′ end of the oligodeoxyribonucleotide (I) are complementary to t-PA cDNA nucleotides 408-397 inclusive and the 12 bases at the 3′ end of the oligodeoxyribonucleotide (I) are complementary to the t-PA cDNA nucleotides 387-376 inclusive.

The oligonucleotide of sequence (I) can be used to delete the portion cDNA which codes for amino acids 67 to 69 of t-PA. It is believed that the cDNA obtained following mutagenesis is similar to that of Pennica et al, 1983, Nature, 301, 214 with the following new nucleotide sequence:-

```
5' - CAG CAG GCC CTG GAT TTC GTG TGC 3'
         |              |  |          |
position 376          387 397        408
```

The t-PA obtained by expression of this cDNA is expected to have a similar amino acid sequence to that described in Pennica et al., except for the following alteration:

```
position 63              66 70              73
        -gln-gln-ala-leu-asp-phe-val-cys-
```

The oligodeoxyribonucleotide employed in variant (b) of the preferred embodiment is suitably at least 16 nucleotides in length, preferably at least 32 nucleotides in length. Suitably it contains at least 8 and preferably at least 16 nucleotides which are complementary to nucleotides on each side of the deletion region.

A preferred oligodeoxyribonucleotide is of sequence (II).

5′d(GGCACACGAAATCTGACAGGGCCTGCTGGCAG)3′ (II)

The 16 bases at the 5′ end of the oligodeoxyribonucleotide (II) are complementary to t-PA cDNA nucleotides 409-394 inclusive and the 16 bases at the 3′ end of the oligodeoxyribonucleotide (II) are complementary to the t-PA cDNA nucleotides 387-372 inclusive.

The oligonucleotide of sequence (II) can be used to delete the portion of cDNA which codes for amino acids 67 and 68 of t-PA. It is believed that the cDNA obtained following mutagenesis is similar to that of Pennica et al,

1983, Nature, 30l, 2l4 with the following new nucleotide sequence:-

```
5' - CAG CAG GCC CTG TCA GAT TTC GTG 3'
       |              | |              |
position 376         387 394          405
```

The oligodeoxyribonucleotides of sequence (I) and (II) are novel and as such form part of the invention.

The modified t-PA of the invention comprises the B chain of native t-PA linked to t-PA A-chain modified in the region of amino acids 67 to 69. This modified t-PA A-chain may be employed as one chain of a fibrinolytically active hybrid protein such as disclosed in EP-0l55387. The modified t-PA A-chain, DNA encoding the modified t-PA A-chain and a hybrid protein comprising the modified t-PA A-chain linked to the B-chain of a fibrinolytically active protease, the catalytic site of which is optionally blocked by a removable blocking group, all form part of the invention. The modified t-PA A-chain may be separated from the B-chain thereof by mild reduction. Alternatively the modified A-chain may be prepared by expressing DNA encoding therefor in a recombinant host cell and recovering the modified A-chain product. The hybrid protein comprising the modified t-PA A-chain linked to the B-chain of a fibrinolytically active protease may be prepared by (a) mixing said chains under oxidative conditions; or (b) ligating DNA encoding said chains and expressing the ligated DNA in a prokaryote or eukaryote host; and thereafter optionally blocking the catalytic site of the hybrid protein with a removable blocking group. The oxidation and reduction conditions are as generally described in EP-0l55387. The hybrid protein may be used in any of the methods and compositions described hereinafter for the modified t-PA itself.

The modified t-PA of the invention is suitably administered in the form of a pharmaceutical composition. Accordingly the present invention also provides a pharmaceutical composition comprising modified t-PA of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile enzyme in sterile isotonic aqueous buffer. Where necessary the composition may also include a solubilising agent to keep the modified t-PA in solution and a local anaesthetic such as lignocaine to ease pain at the site of injection. Generally, the modified t-PA will be supplied in unit dosage form for example as a dry powder or water free concentrate in a hermetically sealed container such as an ampoule or sachette indicating the quantity of protein in activity units. Where the modified t-PA includes a removable blocking group an indication of the time within which the free protein will be liberated may be given. Where the protein is to be administered by infusion, it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration. The quantity of material administered will depend upon the amount of fibrinolysis required and the speed with which it is required, the seriousness of the thromboembolic condition and position and size of the clot. The precise dose to be employed and mode of administration must per force in view of the nature of the complaint be decided according to the circumstances by the physician supervising treatment. However, in general, a patient being treated for a mature, fresh or nascent thrombus will generally receive a daily dose of from 0.0l to l0 mg/kg of body weight either by injection in for example up to five doses or by infusion.

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of modified t-PA of the invention.

The invention also provides a modified t-PA of the invention for use as an active therapeutic substance and, in particular, for use in the treatment of thrombotic diseases.

The following Examples illustrate the invention.

Methods used for Examples 2,3,5 and 6

DNA cleavage

In general the cleavage of about lµg of plasmid DNA or DNA fragments was effected using about 5 units of a restriction enzyme (or enzymes) in about 20µl of an appropriate buffer solution.

Addition of 5' phosphate groups to oligonucleotides:

Addition of either labelled or unlabelled phosphate groups to oligonucleotides was carried out as described (Maxam and Gilbert, Methods in Enzymology Vol. 65 p499-560 Ed. Grossman and Moldane publisher Academic Press l980).

Ligation of DNA Fragments:

Ligation reactions were carried out as described (Maniatis et al Molecular Cloning - A Laboratory Manual, Cold Spring Harbor Laboratory 1982).

Transformation of MI3 DNA

into bacterial cells was carried out using treatment with calcium chloride (Cohen et al., 1973, P.N.A. S. 69, 2110).

Transformation of plasmid DNA

into E. coli HBI0I cells was as described by Hanahan (DNA Cloning Vol I Chapter 6, D.M. Glover ed. IRL Press, 1985) in Protocol 3 except that incubation with RFI was for 5 minutes.

Growth of MI3 single strand DNA:

A single MI3 phage plaque was picked into a 1:100 dilution in 2YT (1.6% Bactotryptone, 1% Yeast extract, 1% NaCl) of a fresh overnight culture of E. coli strain JM 101 (Gronenborn et al., Mol. Gen. Genet. 148, 243-250). The culture was grown at 37° C with shaking for 5-6 hours. The bacteria were pelleted and the supernatant retained. To this was added 200μl of 2.5M NaCl, 20% PEG6000 and the mixture was incubated at room temperature for 15 minutes. The mixture was centrifuged for 5 minutes in an Eppendorf microfuge and the resulting phage pellet was resuspended in 100μl of 10mM Tris pH 7.9, 0.1mM EDTA. After phenol extraction the phage DNA was precipitated with ethanol. The DNA pellet was washed with 70% ethanol, air dried and resuspended in 30μl of 10mM Tris pH 7.9, 0.1mM EDTA.

Growth of double stranded MI3 DNA:

A single MI3 phage plaque was picked into 1ml of 2YT and grown with shaking at 37° C for 6 hours. The bacteria were pelleted and the supernatant containing MI3 phage retained. Meanwhile a one litre 1:100 dilution of an overnight culture of E. coli strain JM 101 was grown at 37° C with shaking for 2 hours. 500μl of the MI3 phage supernatant was added and the culture was shaken at 37° C for a further 4 hours. Preparation of double stranded DNA was carried out as described by Maniatis et al., (Molecular Cloning, A Laboratory Manual, Cold Spring Harbor Laboratory, 1982).

Site directed mutagenesis:

The following double priming mixture was set up in a total volume of 6.9μl (Example 2) or 5.4μl (Example 5) : 0.3 pmoles template DNA (mTR 30 single stranded form, described in EP-0201153-A); 0.60 pmoles kinased mutagenic primer (described in Example I) for Example 2, or 0.44pmoles kinased mutagenic primer (described in Example 4) Example 5; 0.85 pmoles (Example 2) or 0.36pmoles (Example 5) kinased MI3 specific primer (Collaborative Research Inc., Product No. 20205) ; 0.6μl 10 × seq. buffer (0.1M Tris-Cl pH 7.5, 0.05M mgCl2). The mixture was heated in an Eppendorf tube at 56° C for 6 minutes and then allowed to cool to room temperature for at least 15 minutes. To the priming mixture was added 6μl of the following mixture:- 1μl of 10mM solution of dATP, dGTP, dCTP, dTTP, (4μl total) ; 2μl 25mM rATP; 2μl 10 × seq. buffer; 12μl H2O; 2 units T4 DNA ligase for Example 2 or 10 units for Example 5; 2 units DNA Polymerase I (Klenow fragment). The mixture was incubated for 5h at 14° C.

The DNA was transformed in 1μl aliquots into E. coli strain BMH 71-18 mut L (Kramer et al, 1984, Cell 38, 879-887). The transformed bacteria were plated onto a lawn formed from E. coli strain JM 101. Some of the plaques resulting from the transformations were picked and plated to form duplicate gridded arrays of bacterial colonies. These were lifted onto nitrocellulose and lysed as described (Grunstein and Hogness, 1975, P.N.A.S. 72, 3961).

Screening conditions:

The nitrocellulose filters were prehybridised for 2h at 30° C in 6 × SSC (1 × SSC is 150mM NaCl, 15mM tri-sodium citrate) 0.1% SDS, 10 × Denhardt's (Denhardt's is 0.02% Polyvinyl-pyrrolidone, 0.02% Ficoll, 0.02% BSA), 100μg/ml heat denatured, sonicated salmon sperm DNA. The filters were then mixed with a radioactively labelled oligonucleotide of sequence 5'd(ACGAAATCCAGGGCCT)3' (Example 2) or 5'd (GAAATCTGA-CAGGGCCT)3' (Example 5), a shortened version of the mutagenic primer, under the same buffer conditions and were hybridised overnight at 30° C.

The filters were washed at two different temperatures in 6 × SCC plus 0.1% SDS: 3 × 5 minutes at 37° C and 5 minutes at 47° C (Example 2) or 45° C (Example 5). After each wash the filters were exposed, wet (with an intensifying screen) to Fuji RX-100 X-ray film.

Sequencing:

DNA sequencing was carried out by the dideoxy termination method (Sanger, Nicklen and Coulson, 1977, P.N.A.S. 74, 5463).

Plasmid preparation:

Large scale preparation of plasmid DNA and plasmid mini-preparations were carried out as described in Maniatis et al., (Molecular Cloning - A Laboratory Manual, Cold Spring Harbor Laboratory, 1982).

Isolation of DNA fragments from low-melting-point (LMP) agarose gels

DNA fragments were isolated from LMP agarose gels as described by Maniatis et al., (Molecular Cloning, A Laboratory Manual, 1982, Cold Spring Harbor).

Fibrin/agar overlay for detection of t-PA expression

Cell preparation: cells were trypsinised and plated out at $1.35 \times 10^5$ cells per 35mm dish and left overnight in growth medium (10% Serum, 1% stock solution of penicillin/streptomycin; Flow Laboratories, 1% Glutamine, 1% stock solution of non-essential amino acids; Flow Laboratories, in Eagles MEM) at 37°C in a humidified incubator in an atmosphere of 5% $CO_2$/95% air.

Transfection procedure:

The transfection used (calcium coprecipitation) is described in 'DNA Cloning' Ed. D.M. Glover (Chap. 15, C. Gorman). Glycerol shock and 10mM butyrate treatment were used. Following transfection the cells were rested overnight in growth medium.

Overlay:

Agarose (Indubiose $A_{37}$), 2.4g in 95ml Eagles MEM (Gibco) heated to melting point in a bunsen flame, was then placed in a water bath maintained at 48°C. 5.6 ml of fibrinogen (20mg/ml) were diluted 1:1 with 5.6 ml of Eagles MEM (containing an extra 7 mg/ml of NaCl) and retained on ice. 3.3 ml of Eagles MEM (no additions) were aliquoted into a bijou containing 86μl of bovine thrombin at 50 NIH Units/ml (retained on ice). The cells were washed 3 times with Eagles MEM to remove serum. The thrombin and fibrinogen were warmed to 37°C in a water bath. 9.5 ml agarose were aliquoted into a pre-warmed universal. The thrombin was added to the agar, followed by the fibrinogen. The gel was poured over the cell layer and incubated at 37°C until lysis zones appeared.

Example 1

5'd (GCACACGAAATCCAGGGCCTGCTG)3'

The above mentioned 24-mer was prepared on an Applied Biosystems 381A automated DNA synthesiser according to the manufacturer's instructions.

Example 2

Oligodeoxyribonucleotide site-directed mutagenesis to produce novel DNA coding for des (tyr$_{67}$-ser$_{69}$)t-PA

The oligodeoxyribonucleotide prepared in Example 1 was used to produce DNA coding for a novel modified t-PA protein.

The oligodeoxyribonucleotide is complementary to the nucleotides 376-387 and 397-408 inclusive of the t-PA cDNA (Pennica et al., Nature, 1983, 301, 214-221). The nucleotides complementary to 388-396 inclusive are not present in this oligodeoxyribonucleotide and its use in site-directed mutagenesis is intended to delete 3 codons from the t-PA coding sequence.

The site directed mutagenesis and DNA sequencing was carried out as described above in Methods.

The nucleotides up to position 387 in the mutant DNA have been shown to be adjacent to nucleotides 397 onwards of the t-PA cDNA sequence. This sequencing data has confirmed that the nucleotides 388-396 have been deleted from the t-PA gene.

Example 3

Expression of des (tyr$_{67}$-ser$_{69}$) t-PA in Eukaryotic Cells

The approx. 2kb BglII fragment encoding the mature modified t-PA of Example 2 or unmodified t-PA was excised from the appropriate M13 construct, and cloned into the unique BglII site of the expression vector pTRE12 (described in EP-0201153-A) using standard restriction endonuclease digestion and ligation techniques described in 'Molecular Cloning - A Laboratory Manual' by T. Maniatis, E.F. Fritsch and J. Sambrook, Cold Spring Harbor Laboratory (1982). Recombinant plasmids grown in E. Coli HB101 were characterised by restriction endonuclease digestion and mapping to identify isolates carrying the BglII fragment in the correct orientation for expression; these were called pTRE15 (unmodified t-PA) or pTRE53 (modified t-PA). Plasmid DNA prepared from recombinant E. coli HB101 by standard techniques was used to transfect cultured mouse L929 cells by the calcium phosphate precipitation technique essentially as described in 'High Efficiency Gene Transfer into Mammalian Cells' by C. Gorman (In 'DNA Cloning Volume II', ed. D. Glover, IRL Press Ltd., 1985).

Expression of modified and unmodified t-PA from transfected mouse L929 cells (24 hours post-transfection) was detected using the fibrin/agarose overlay technique essentially as described by Jones et al., Cell, 1975, 5, 323-329. Lytic zones were obtained from pTRE53 (modified t-PA) transfected cells. In a similar experiment lytic zones were obtained from PTRE15 (unmodified t-PA) but not from cells transfected with the empty parent vector pTRE12 (t-PA-).

The protein produced in the transient expression system was also examined by fibrin zymography.

Following butyrate treatment the cell monolayers were washed with serum-free harvest medium and then incubated for 24h in serum-free medium. A sample of medium from each culture was then subjected to fibrin zymography as described in Dodd et al., (1986), Thrombosis and Heamostasis 55 (I); 94. The molecular weights of the species of activator present in pTRE53 harvest medium (modified t-PA) appear to be indistinguishable from those present in pTREI5 harvest medium (unmodified t-PA), as would be expected for such a small deletion.

## Example 4

5′d(GGCACACGAAATCTGACAGGGCCTGCTGGCAG)3′

The above mentioned 32-mer was prepared analogously to the compound of Example I.

## Example 5

Oligodeoxyribonucleotide site-directed mutagenesis to produce novel DNA coding for des(tyr$_{67}$-phe$_{68}$)t-PA

The oligodeoxyribonucleotide prepared in Example 4 was employed in a site directed mutagenesis as described above in Methods.

DNA sequencing (see Methods) of the mutant DNA revealed that the nucleotides 388-393 have been deleted from the t-PA gene.

## Example 6

Expression of des (tyr$_{67}$-phe$_{68}$)t-PA in Eukaryotic cells

The BglII fragment encoding the mature modified t-PA of Example 5 was inserted into vector PTREI2 by an analogous procedure to that of Example 3, to yield plasmid PTRE54. Expression of the plasmid PTRE54 in mouse L929 cells was carried out by an analogous procedure to that of Example 3. Lytic zones were obtained from pTRE54 transfected cells. The protein product was examined by fibrin zymography as described in Example 3. The molecular weights of the species of activator present in pTRE54 harvest medium appear to be indistinguishable from those present in pTREI5 harvest medium, as would be expected for such a small deletion.

## Claims

I. A fibrinolytically active tissue-type plasminogen activator which has been modified in the region of amino acids 67 to 69.

2. A modified tissue-type plasminogen activator according to claim I, wherein the modification comprises the deletion of all or part of said region.

3. A modified tissue-type plasminogen activator according to claim I or 2, wherein the modification comprises the deletion of amino acid residues 67 and 68 or 67 to 69.

4. A modified tissue-type plasminogen activator prepared according to Example 3 or 6.

5. A derivative of the modified tissue-type plasminogen activator according to any of claims I to 4.

6. A DNA polymer comprising a nucleotide sequence that encodes a modified tissue-type plasminogen activator according to any of claims I to 4.

7. A replicable expression vector capable, in a host cell, of expressing a DNA polymer according to claim 6.

8. A host cell transformed with the vector according to claim 7.

9. An oligodeoxyribonucleotide comprising a nucleotide sequence which is complementary or corresponds to a region of the t-PA cDNA extending 5′ from nucleotide 387 inclusive, said sequence being joined directly to a nucleotide sequence which is complementary or corresponds to a region extending 3′ from nucleotide 397 or 394 inclusive of t-PA cDNA.

I0. An oligodeoxyribonucleotide having the sequence (I) or (II):

5′d(GCACACGAAATCCAGGGCCTGCTG)3′     (I)

or

5′d(GGCACACGAAATCTGACAGGGCCTGCTGGCAG)3′     (II)

II. t-PA A-chain which has been modified in accordance with any of claims I to 4.

I2. A DNA polymer comprising a nucleotide sequence that encodes the modified t-PA A-chain according to claim II.

I3. A hybrid protein comprising the modified t-PA A-chain according to claim II linked to the B-chain of a fibrinolytically active protease, the catalytic site of which is optionally blocked by a removable blocking group.

I4. A pharmaceutical composition comprising modified tissue-type plasminogen activator according to any of claims I to 5 in combination with a pharmaceutically acceptable carrier.

I5. A modifies tissue-type plasminogen activator according to any of claims I to 5 for use as an active therapeutic substance.

I6. A modified tissue-type plasminogen activator according to any of claims I to 5 for use in the treatment of thrombotic disease.

I7. Use of a modified tissue-type plasminogen activator according to any of claims I to 5 for the preparation of a medicament for the treatment of thrombotic disease.

I8. A process for preparing modified tissue-type plasminogen activator according to claim I, which process comprises expressing DNA encoding said modified tissue-type plasminogen activator in a recombinant host cell and recovering the modified tissue-type plasminogen activator product.

I9. Modified tissue-type plasminogen activator obtainable by the process of claim I8.

Claims for the following Contracting State : ES

I. A process for preparing a fibrinolytically active tissue-type plasminogen activator which has been modified in the region of amino acids 67 to 69, which process comprises expressing DNA encoding said modified tissue-type plasminogen activator in a recombinant host cell and recovering the modified tissue-type plasminogen activator product.

2. A process according to claim I, wherein the modification comprises the deletion of all or part of said region.

3. A process according to claim I or 2, wherein the modification comprises the deletion of amino acid residues 67 and 68 or 67 to 69 inclusive.

4. A process for preparing a DNA polymer comprising a nucleotide sequence that encodes a modified tissue-type plasminogen activator according to any of claims I to 3, by the condensation of appropriate mono-, di- or digomeric nucleotide units.

5. A process for preparing a replicable expression vector capable, in a host cell, of expressing a DNA polymer as defined in claim 4, which process comprises cleaving a vector compatible with said host cell to provide a linear DNA segment having an intact replicon, and combining said linear segment with one or more DNA molecules which, together with said linear segment, encode the modified t-PA, under ligating conditions.

6. A process for preparing a host cell transformed with the vector as defined in claim 5, which process comprises transforming a host cell with a said vector under transforming conditions.

7. A process for preparing an oligodeoxyribonucleotide comprising a nucleotide sequences which is complementary or corresponds to a region of the t-PA cDNA extending 5′ from nucleotide 387 inclusive, said sequence being joined directly to a nucleotide sequence which is complementary or corresponds to a region extending 3′ from nucleotide 397 or 394 inclusive of t-PA cDNA, which process comprises the deprotection of a second oligodeoxyribonucleotide having the same sequence of bases as said oligodeoxyribonucleotide and in which all free hydroxy and amino groups are protected.

8. A process according to claim 4, which process comprises effecting a site-directed mutagenesis upon the cDNA which codes for tissue-type plasminogen activator.

9. A process according to claim 8, wherein the site-directed mutagenesis is effected using an oligodeoxyribonucleotide as defined in claim 7.

I0. A process for preparing t-PA A-chain which has been modified in accordance with any of claims I to 3, which process comprises separating the A-chain of t-PA which has been so modified from the B-chain thereof by mild reduction.

II. A process for preparing t-PA A-chain as defined in claim I0, which process comprises expressing DNA encoding said modified A-chain in a recombinant host cell and recovering the modified t-PA A-chain product.

I2. A process for preparing a hybrid protein comprising the modified t-PA A-chain as defined in claim I0 linked to the B-chain of a fibrinolytically active protease, the catalytic site of which is optionally blocked by a removable blocking group, which process comprises mixing said A- and B- chains under oxidative conditions and thereafter optionally blocking the catalytic site with a removable blocking group.

I3. A process for preparing a protein as defined in claim I2, which process comprises ligating DNA encoding said A-chain to DNA encoding said B-chain, expressing the ligated DNA in a prokaryote or eukaryote host and thereafter optionally blocking the catalytic site of the expressed product with a removable blocking group.

I4. Use of a modified tissue-type plasminogen activator according to any of claims I to 3 for the preparation of a medicament for the treatment of thrombotic disease.

I5. A fibrinolytically active tissue-type plasminogen activator as defined in claim I, whenever prepared by the process of claim I or an obvious equivalent thereof.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 093 619  (GENENTECH, INC.)<br>* Page 9, lines 19-33; claims * | 1 | C 12 N  15/00<br>C 12 N   9/64<br>A 61 K  37/54 |
| D,Y | PROCEEDINGS OF THE NATIONAL ACADEMY OF SCIENCES OF THE USA, vol. 81, no. 17, September 1984, pages 5355-5359, Washington, US; T. NY et al.: "The structure of the human tissue-type plasminogen activator gene: correlation of intron and exon structures to functional and structural domains"<br>*  Introduction; pages 5358, column 1, line 18 - page 5359,  column 1, line 28 * | 1 | |
| D,A | FEBS LETTERS, vol. 163, no. 1, October 1983, pages 37-41, Elsevier Science Publishers B.V., Amsterdam, NL; L. BANYAI et al.: "Common evolutionary origin of the fibrin-binding structures of fibronectin and tissue-type plasminogen activator"<br>*  Introduction;  page 38, column 2, lines 10-19; page  39,  column 1, lines 3'-8; figures 1a,2;  * | 1 | **TECHNICAL FIELDS SEARCHED (Int. Cl.4)**<br><br>C 12 N |

--- -/-

The present search report has been drawn up for all claims

| Place of search | Date of completion of the search | Examiner |
|---|---|---|
| THE HAGUE | 07-07-1987 | YEATS S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

| | **DOCUMENTS CONSIDERED TO BE RELEVANT** | | Page 2 |
|---|---|---|---|

| Category | Citation of document with indication, where appropriate, of relevant passages | Relevant to claim | CLASSIFICATION OF THE APPLICATION (Int. Cl.4) |
|---|---|---|---|
| P,X | EP-A-0 207 589  (BEECHAM GROUP PLC) <br> * Page 1, lines 1-39; page 16, lines 20-36; claims * <br><br> ----- | 1-8,11 -19 | |
| | | | TECHNICAL FIELDS SEARCHED (Int. Cl.4) |
| | The present search report has been drawn up for all claims | | |

| Place of search <br> THE HAGUE | Date of completion of the search <br> 07-07-1987 | Examiner <br> YEATS S.M. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82